# EUROPÄISCHE PATENTSCHRIFT

(11) **EP 1 641 415 B1**
(45) Veröffentlichungstag und Bekanntmachung des Hinweises auf die Patenterteilung: **19.09.2007**
(21) Anmeldenummer: 04740358.9
(22) Anmeldetag: 26.06.2004
(51) Int. Cl.: A61F 6/00

(54) **ZWISCHENWIRBELIMPLANTAT**
INTERVERTEBRAL IMPLANT
IMPLANT INTERVERTEBRAL

(30) Priorität: 08.07.2003 DE 10330698
(43) Veröffentlichungstag der Anmeldung: 05.04.2006
(73) Patentinhaber: Aesculap AG & Co. KG, 78532 Tuttlingen (DE)
(72) Erfinder: BLÖMER, Wilhelm, 88690 Unteruhldingen-Mühlhofen (DE); SCHULTZ, Robert, 78532 Tuttlingen (DE)
(74) Vertreter: Boehme, Ulrich
(86) Internationale Anmeldenummer: PCT/EP2004/006956
(87) Internationale Veröffentlichungsnummer: WO 2005/004747

(56) Entgegenhaltungen:
- EP-A- 0 560 140
- WO-A-03/075804
- US-A- 5 258 031
- US-A- 5 888 226

## Beschreibung

Die Erfindung betrifft ein Zwischenwirbelimplantat mit zwei Tragplatten zur Anlage an benachbarten Wirbelkörpern mit einer konvexen, balligen Gelenkfläche an einer Tragplatte und einer konkaven, balligen Gelenkfläche an der anderen Tragplatte, wobei die Gelenkflächen flächig aneinander anliegen und dadurch die Tragplatten schwenkbar aneinander abstützen und wobei der höchste beziehungsweise tiefste Punkt der Gelenkflächen zwischen der Hinterkante und der Mitte der Tragplatten positioniert ist.

Ein solches Zwischenwirbelimplantat ist beispielsweise aus der US-Patentschrift 5,258,031 A sowie der WO 95/26697 A1 bekannt. Die Verlagerung des höchsten Punktes und des niedrigsten Punktes der balligen Gelenkflächen, also der Ebene, in der sich der Mittelpunkt der balligen Gelenkflächen befindet, in Richtung auf die Hinterkante des Implantates entspricht den anatomischen Gegebenheiten, benachbarte Wirbelkörper verschwenken bei ihrer gegenseitigen Verschwenkbewegung um einen Drehpunkt, der zwischen der Mitte und dem dorsalen Ende der Bandscheibe liegt, und dementsprechend ist bei der vorbekannten Prothese das Kugelgelenk im dorsalen Drittel der Bandscheibe positioniert. Allerdings hat dies bei der bekannten Anordnung zur Folge, daß die Abstützkräfte über die relativ kleinen balligen Gelenkflächen ausschließlich im hinteren Drittel auf die Tragplatten übertragen werden, diese Tragplatten können somit die Abstützkräfte nicht gleichmäßig auf die Wirbelkörper übertragen, sondern die Abstützkräfte werden im wesentlichen im hinteren Drittel auftreten. Insbesondere bei geschwächten Wirbelkörpern kann dies zu unerwünschten Einbrüchen der Tragplatten im dorsalen Wirbelkörperbereich führen.

In der US 5,534,029 A ist ein Zwischenwirbelimplantat beschrieben, bei dem die Gelenkflächen exakt mittig zwischen Vorderkante und Hinterkante angeordnet sind, bei diesem Zwischenwirbelimplantat ist also die anatomisch wünschenswerte Verlagerung des Drehpunktes in dorsaler Richtung nicht realisiert.

Es ist Aufgabe der Erfindung, ein gattungsgemäßes Zwischenwirbelimplantat so zu verbessern, daß auch bei einer Verlagerung des Drehpunktes des Zwischenwirbelimplantats in dorsaler Richtung eine über den Wirbelkörper gleichmäßige Krafteinleitung erreicht wird.

Diese Aufgabe wird bei einem Zwischenwirbelimplantat der eingangs beschriebenen Art erfindungsgemäß dadurch gelöst, daß sich die konvexe Gelenkfläche im mittleren Bereich der Tragplatte zu beiden Seiten der zwischen Vorderkante und Hinterkante verlaufenden Mittellinie der Tragplatte etwa gleich weit erstreckt und an seinem der Hinterkante zugewandten Ende weiter von der Tragplatte absteht als an seinem der Vorderkante zugewandten Ende, derart, daß die konvexe Gelenkfläche einerseits mittig in der Tragplatte angeordnet ist und andererseits einen Drehpunkt bildet, der zur Hinterkante verschoben ist, und daß die Gelenkflächen aus Keramik bestehen. Damit erhält man eine Gelenkfläche, die mittig in der Tragplatte angeordnet ist und trotzdem einen Drehpunkt bildet, der in dorsaler Richtung, also zur Hinterkante hin, verschoben ist. Die Krafteinleitung kann über die gesamte Gelenkfläche etwa mittig erfolgen, so daß die Gefahr einer einseitigen Belastung der Tragplatten deutlich herabgesetzt ist. An sich ist ein Zwischenwirbelimplantat mit in dorsaler Richtung verlagertem Drehpunkt und mit einer Gelenkfläche, die an der Hinterkante weiter von der Tragplatte absteht als an der Vorderkante, bereits in der nicht vorveröffentlichten WO 03/075804 A1 beschrieben, jedoch sind die Gleitflächen bei dieser bekannten Ausgestaltung durch eine Paarung von Metall einerseits und einem Kunststoff andererseits gebildet, so daß die Bildung von Abrieb nicht ausgeschlossen werden kann.

Besonders vorteilhaft ist es, wenn mindestens eine der Gelenkflächen in einem Einsatz angeordnet ist, der in der Tragplatte festgelegt ist, insbesondere können beide Gelenkflächen in derartigen Einsätzen angeordnet sein.

Es ist vorteilhaft, wenn diese Einsätze in eine Ausnehmung der Tragplatten eingesetzt sind, so daß sie dadurch gegen eine seitliche Verschiebung gesichert werden.

Die konvexe Gelenkfläche kann an ihrem der Hinterkante zugewandten Ende in eine hintere, senkrechte Abschlußfläche übergehen.

Ebenso kann vorgesehen sein, daß die konkave Gelenkfläche an ihrem der Vorderkante zugewandten Ende in eine vordere senkrechte Abschlußfläche übergeht. Diese Abschlußflächen liegen dann vorzugsweise an den Seitenwänden der Ausnehmung an und zentrieren den Einsatz.

Die konkave Gelenkfläche reicht dabei insbesondere bis an das der Hinterkante der Tragplatte zugewandte Ende des Einsatzes heran. Sie ist also in einem mittig in die Tragplatte eingesetzten Einsatz außermittig angeordnet, das heißt in Richtung auf die Hinterkante verschoben.

Günstig ist es, wenn in der Tragplatte mit der konvexen Gleitfläche zwischen dieser und der Vorderkante der Tragplatte eine Vertiefung angeordnet ist. Diese ermöglicht es, bei extremen Verschwenkwinkeln vorstehende Teile an der anderen Tragplatte aufzunehmen, so daß dadurch der Verschwenkwinkel erhöht werden kann.

Die Gleitflächen können bei einer anderen Ausführungsform auch einstückig mit den Tragplatten ausgebildet sein.

Bei der Anordnung der Gleitflächen in einem Einsatz wird üblicherweise der gesamte Einsatz aus Keramik gefertigt, bei der einstückigen Ausgestaltung der Tragplatten werden Tragplatten mit entsprechenden Gelenkflächen aus Keramik gefertigt.

Die nachfolgende Beschreibung bevorzugter Ausführungsformen der Erfindung dient im Zusammenhang mit der Zeichnung der näheren Erläuterung. Es zeigen:

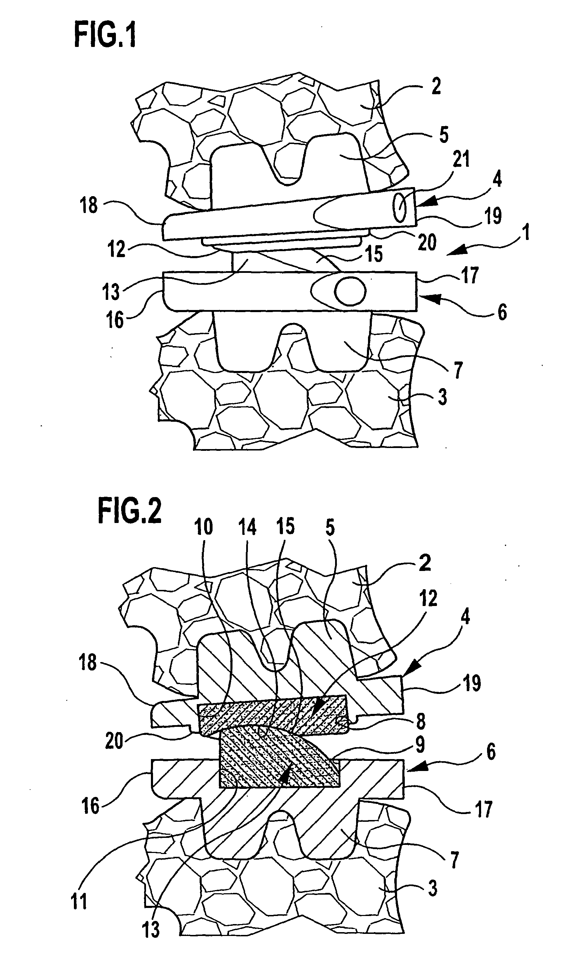

- Figur 1:: eine Seitenansicht eines Zwischenwirbelimplantats;
- Figur 2:: eine Längsschnittansicht des Zwischenwirbelimplantats der Figur 1;
- Figur 3:: eine perspektivische Draufsicht auf eine der beiden Tragplatten des Implantats der Figur 1 und
- Figur 4:: eine perspektivische Draufsicht auf die andere Tragplatte des Implantats der Figur 1.

Das in der Zeichnung dargestellte Zwischenwirbelimplantat 1 dient als Ersatz einer entfernten Bandscheibe und wird zwischen zwei Wirbelkörper 2, 3 eingesetzt. Es umfaßt eine erste Tragplatte 4, die auf ihrer Rückseite rippenförmige Vorsprünge 5 zur Verankerung in einem Wirbelkörper trägt sowie eine zweite Tragplatte 6, die ebenfalls auf ihrer Rückseite entsprechende Vorsprünge 7 zur Verankerung im benachbarten Wirbelkörper trägt. Die einander zugewandten Innenseiten der Tragplatten 4 und 6 sind eben ausgebildet und verlaufen im implantierten Zustand im wesentlichen parallel zueinander.

Beide Tragplatten 4, 6 weisen auf ihrer Innenseite eine zentrale Ausnehmung 8 beziehungsweise 9 auf mit senkrechten Seitenwänden 10 beziehungsweise 11 und mit einer im wesentlichen ovalen Fläche. In beide Ausnehmungen 8, 9 ist jeweils ein Gelenkeinsatz 12 beziehungsweise 13 aus Keramik dauerhaft eingesetzt. In den einen Gelenkeinsatz 12 ist eine konkave, ballige Gelenkfläche 14 eingearbeitet, die Oberseite des anderen Gelenkeinsatzes 13 ist als ballige, konvexe Gelenkfläche 15 geformt. Dabei sind die Gelenkflächen als Kugelteilflächen ausgebildet und komplementär zueinander geformt, so daß sie flächig aneinander anliegen und dadurch eine verschwenkbare Lagerung der beiden Tragplatten 3, 4 ausbilden.

Die konvexe Gelenkfläche 15 erstreckt sich über die gesamte Oberseite des Gelenkeinsatzes 13, der höchste Punkt der Gelenkfläche 15 ist dabei in Richtung auf die Hinterkante 16 der Tragplatte 6 verlagert, befindet sich also nicht in der Mitte des Gelenkeinsatzes 13, der aber seinerseits mittig zwischen der Hinterkante 16 und der Vorderkante 17 der Tragplatte 6 angeordnet ist. Dadurch ist der Gelenkeinsatz 13 an seinem hinterkantenseitigen Ende dicker als am vorderkantenseitigen Ende, die Gelenkfläche 15 taucht am vorderkantenseitigen Ende bis in die Ausnehmung 9 ein.

Auch die konkave Gelenkfläche 14 ist in dem mittig in die Tragplatte 4 eingesetzten Gelenkeinsatz 12 in Richtung auf die Hinterkante 18 dieser Tragplatte 4 verlagert, während der Gelenkeinsatz 12 mittig zwischen dieser Hinterkante 18 und der Vorderkante 19 der Tragplatte 4 angeordnet ist. Auf diese Weise erhält man eine Verlagerung des Verschwenkpunktes in Richtung auf die Hinterkante 18, also in den dorsalen Teil des Zwischenwirbelimplantats 1, und trotzdem werden die Abstützkräfte großflächig und weitgehend zentral in die Tragplatten 4, 6 eingeleitet, so daß diese zentral belastet werden und diese Belastung symmetrisch über die gesamte Abstützfläche an die benachbarten Wirbelkörper 2, 3 übertragen können.

Der Gelenkeinsatz 13 mit der konvexen Gelenkfläche 15 ist in eine Ausnehmung 9 eingesetzt, die unmittelbar in die Innenfläche der Tragplatte 6 übergeht, dagegen wird die Ausnehmung 8 der anderen Tragplatte 4, die den Gelenkeinsatz 12 mit der konkaven Gelenkfläche 14 aufnimmt, von einer Ringschulter 20 umgeben, die etwa bis zur halben Höhe des aus der Ausnehmung 8 hervorstehenden Gelenkeinsatzes 12 von der Innenseite der Tragplatte 4 absteht und dicht am Gelenkeinsatz 12 anliegt, der dadurch über eine größere Höhe eine Abstützung in der Tragplatte 4 erfährt.

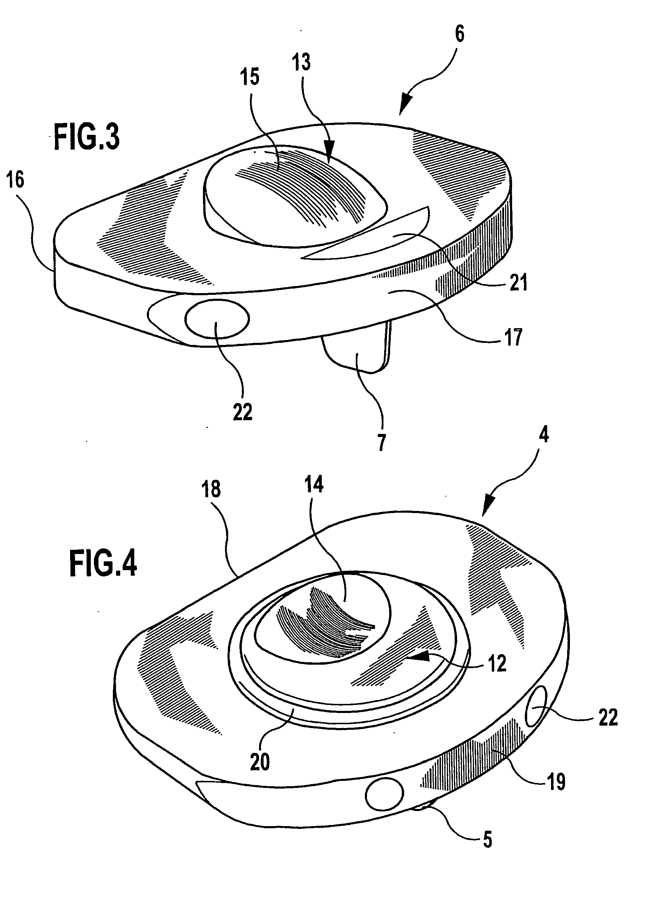

In der Tragplatte 6 befindet sich zwischen der Ausnehmung 9 und der Vorderkante 17 eine Vertiefung 21 (Figur 3), in die beim Verschwenken der Tragplatten gegeneinander die Ringschulter 20 eintauchen kann, dadurch läßt sich der maximale Verschwenkwinkel geringfügig vergrößern.

In beiden Tragplatten 4, 6 sind Einstecköffnungen 22 vorgesehen, in die ein Handhabungs- und Einsetzwerkzeug eingesetzt werden kann, mit dessen Hilfe das Zwischenwirbelimplantat 1 in den Zwischenwirbelraum zwischen die beiden Wirbelkörper 2, 3 eingesetzt wird.

## Patentansprüche

1. Zwischenwirbelimplantat mit zwei Tragplatten zur Anlage an benachbarten Wirbelkörpern mit einer konvexen, balligen Gelenkfläche an einer Tragplatte und einer konkaven, balligen Gelenkfläche an der anderen Tragplatte, wobei die Gelenkflächen flächig aneinander anliegen und **dadurch** die Tragplatten schwenkbar aneinander abstützen und wobei der höchste beziehungsweise tiefste Punkt der Gelenkflächen zwischen der Hinterkante und der Mitte der Tragplatten positioniert ist, **dadurch gekennzeichnet, daß** sich die konvexe Gelenkfläche (15) im mittleren Bereich der Tragplatte (6) zu beiden Seiten der zwischen Vorderkante (17) und Hinterkante (16) verlaufenden Mittellinie der Tragplatte etwa gleich weit erstreckt und an seinem der Hinterkante (16) zugewandten Ende weiter von der Tragplatte (6) absteht als an seinem der Vorderkante (17) zugewandten Ende, derart, daß die konvexe Gelenkfläche (15) einerseits mittig in der Tragplatte angeordnet ist und andererseits einen Drehpunkt bildet, der zur Hinterkante (15) verschoben ist, und daß die Gelenkflächen (14, 15) aus Keramik bestehen.

2. Zwischenwirbelimplantat nach Anspruch 1, **dadurch gekennzeichnet, daß** mindestens eine der Gelenkflächen (14, 15) in einem Einsatz (12 beziehungsweise 13) angeordnet ist, der an der Tragplatte (4 beziehungsweise 6) festgelegt ist.

3. Zwischenwirbelimplantat nach Anspruch 2, **dadurch gekennzeichnet, daß** der Einsatz (12, 13) in eine Ausnehmung (8 beziehungsweise 9) der Tragplatte (4 beziehungsweise 6) eingesetzt ist.

4. Zwischenwirbelimplantat nach einem der voranstehenden Ansprüche, **dadurch gekennzeichnet, daß** die konvexe Gelenkfläche (15) an ihrem der Hinterkante (16) zugewandten Ende in eine hintere senkrechte Abschlußfläche übergeht.

5. Zwischenwirbelimplantat nach einem der voranstehenden Ansprüche, **dadurch gekennzeichnet, daß** die konvexe Gelenkfläche (15) an ihrem der Vorderkante (17) zugewandten Ende in eine vordere senkrechte Abschlußfläche übergeht.

6. Zwischenwirbelimplantat nach einem der Ansprüche 2 bis 5, **dadurch gekennzeichnet, daß** die konkave Gelenkfläche (14) bis an das der Hinterkante (18) der Tragplatte (4) zugewandte Ende des Einsatzes (12) heranreicht.

7. Zwischenwirbelimplantat nach einem der voranstehenden Ansprüche, **dadurch gekennzeichnet, daß** in der Tragplatte (6) mit der konvexen Gelenkfläche (15) zwischen dieser und der Vorderkante (17) eine Vertiefung (21) angeordnet ist.

8. Zwischenwirbelimplantat nach Anspruch 1, **dadurch gekennzeichnet, daß** die Gelenkflächen (14, 15) einstückig mit den Tragplatten (4; 6) ausgebildet sind.

## Claims

1. An intervertebral implant with two supporting plates which lie against adjacent vertebrae, with a convex spherical joint surface on one supporting plate and a concave spherical joint surface on the other supporting plate, wherein the joint surfaces lie flat against one another and the supporting plates are thereby pivotably supported against one another and wherein the highest and deepest points of the joint surfaces are positioned between the rear edge and the middle of the supporting plates, **characterised in that** the convex joint surface (15) in the middle region of the supporting plate (6) extends approximately equally far on both sides of the centre line of the supporting plate, said centre line extending between the front edge (17) and the rear edge (16), and projects further from the supporting plate (6) at its end facing the rear edge (16) than at its end facing the front edge (17) so that the convex joint surface (15) on the one hand is arranged in the middle of the supporting plate and on the other hand forms a pivot which is displaced towards the rear edge (15)¹, and **in that** the joint surfaces (14, 15) are made of ceramic.
¹ The reference numeral should be (16).

2. An intervertebral implant according to claim 1, **characterised in that** at least one of the joint surfaces (14, 15) is arranged in an insert (12 or 13) secured to the supporting plate (4 or 6).

3. An intervertebral implant according to claim 2, **characterised in that** the insert (12, 13) is inserted into a recess (8 or 9) in the supporting plate (4 or 6).

4. An intervertebral implant according to any one of the preceding claims, **characterised in that** the convex joint surface (15) merges into a rear vertical boundary surface at its end facing the rear edge (16).

5. An intervertebral implant according to any one of the preceding claims, **characterised in that** the convex joint surface (15) merges into a front vertical boundary surface at its end facing the front edge (17).

6. An intervertebral implant according to any one of claims 2 to 5, **characterised in that** the concave joint surface (14) extends as far as that end of the insert (12) which faces the rear edge (18) of the supporting plate (4).

7. An intervertebral implant according to any one of the preceding claims, **characterised in that**, in the supporting plate (6) with the convex joint surface (15), a depression (21) is arranged between the latter and the front edge (17).

8. An intervertebral implant according to claim 1, **characterised in that** the joint surfaces (14, 15) are formed in one piece with the supporting plates (4; 6).

## Revendications

1. Implant intervertébral comprenant deux plaques support pour l'application contre des corps vertébraux adjacents, avec une surface d'articulation sphérique convexe sur une plaque support et une surface sphérique concave sur l'autre plaque support, lesdites surfaces d'articulation reposant l'une sur l'autre et soutenant ainsi les plaques support l'une contre l'autre en permettant leur pivotement, et le point le plus élevé ou le point plus bas des surfaces d'articulation étant positionné entre le bord arrière et le milieu des plaques supports, **caractérisé en ce que** la surface d'articulation convexe (15) s'étend dans la région centrale de la plaque support (6) à distance sensiblement égale des deux côtés de la ligne médiane de la plaque support comprise entre le bord avant (17) et le bord arrière (16), et s'élève plus de la plaque support (6) sur son extrémité dirigée vers le bord arrière (16) que sur son extrémité dirigée vers le bord avant (17), de telle manière que la surface d'articulation convexe (15) est centralement disposée dans la plaque support, d'une part, et qu'elle forme d'autre part un point de rotation décalé vers le bord arrière (16), et **en ce que** les surfaces d'articulation (14, 15) sont en céramique.

2. Implant intervertébral selon la revendication 1, **caractérisé en ce qu'**au moins une des surfaces d'articulation (14, 15) est disposée dans une garniture (12 ou 13) fixée sur la plaque support (4 ou 6).

3. Implant intervertébral selon la revendication 2, **caractérisé en ce que** la garniture (12, 13) est mise en place dans une cavité (8 ou 9) de la plaque support (4 ou 6).

4. Implant intervertébral selon l'une des revendications précédentes, **caractérisé en ce que** la surface d'articulation convexe (15) se termine par une surface terminale arrière verticale sur son extrémité dirigée vers le bord arrière (16).

5. Implant intervertébral selon l'une des revendications précédentes, **caractérisé en ce que** la surface d'articulation convexe (15) se termine par une surface terminale avant verticale sur son extrémité dirigée vers le bord avant (17).

6. Implant intervertébral selon l'une des revendications 2 à 5, **caractérisé en ce que** la surface d'articulation concave (14) s'étend jusqu'à l'extrémité de la garniture (12) dirigée vers le bord arrière (18) de la plaque support (4).

7. Implant intervertébral selon l'une des revendications précédentes, **caractérisé en ce qu'**un évidement (21) est ménagé dans la plaque support (6) avec la surface d'articulation convexe (15), entre celle-ci et le bord avant (17).

8. Implant intervertébral selon la revendication 1, **caractérisé en ce que** les surfaces d'articulation (14, 15) sont réalisées d'un seul tenant avec les plaques support (4 ; 6).
